# FASCICULE DE BREVET EUROPEEN

(11) **EP 0 369 923 B1**
(45) Date de publication et mention de la délivrance du brevet: **15.09.1993**
(21) Numéro de dépôt: 89500110.5
(22) Date de dépôt: 08.11.1989
(51) Int. Cl.: A61F 5/445

(54) **Dipositif obturateur pour le contrôle d'iléo-colostomies**
Verschlusselement zur Kontrolle von Ileokolostomie
Closure device for controlling ileo colostomies

(30) Priorité: 15.11.1988 ES 8803463; 25.10.1989 ES 8903591
(43) Date de publication de la demande: 23.05.1990
(73) Titulaire: Vidal Sans, José, ES-08029 Barcelona (ES)
(72) Inventeur: Vidal Sans, José, ES-08029 Barcelona (ES)
(74) Mandataire: Espiell Volart, Eduardo Maria

(56) Documents cités:
- EP-A- 0 037 785
- EP-A- 0 191 646
- DE-A- 2 634 642
- THE LANCET, vol. II, no. 8515, 8 novembre 1986, pages 1062-1063; FLEMING BURGHARTH et al.: "The colostomy plug: a new disposable device for a continent colostomy"

## Description

La présente invention concerne un dispositif obturateur destiné au contrôle des iléo-colostomies pouvant être utilisé par le patient d'une façon pratique et commode pour réduire les difficultés personnelles que comporte vivre avec une ostomie, notamment le contrôle de la diète, de la médication, des réglages sexuels, de l'hygiène personnelle et de certains problèmes sociaux. Comme l'on sait, sur le marché des articles chirurgiques il y a un dispositif courant adopté par de nombreux opérés auxquels on a pratiqué un anus contre nature, un dispositif consistant en un anneau qui est fixé à la paroi abdominale extérieure et auquel est appliquée et immobilisée une poche collectrice des évacuations (poche à colostomie).

La technologie actuelle a amélioré cette exécution élémentaire en retirant ladite poche lorsqu'on n'en a pas besoin et en employant, à sa place, un élément de fermeture en manière de bouchon, qui est introduit dans l'ouverture du colon et qui est fixé soit magnétiquement (modèle de fermeture développé par H. Feustel et G. Henning et présenté au Congrès de Chirurgie de Munich de 1975) ou bien simplement pression et en adoptant un obturateur expansif (modèle inventé en 1986 par Flemming Burcharth, Frederik Kylberg, Akell Ballan et Sten Norby Rasmussen - (Depart. of Surgical, Gastroenterology du Danemark, Depart. of Surgery de la Suède et Depart. of Medical Gastroenterology, du Danemark).

Ces exécutions offrent, cependant, plusieurs inconvénients. En effet, la première oblige à ce qu'un anneau magnétique soit cousu à la paroi abdominale, coopérant avec un aimant logé à l'intérieur d'un bouchon. On prévoit également un autre anneau magnétique entourant ledit aimant central. L'insertion de l'anneau à placer autour de l'anus contre nature représente une intervention qui n'est pas libre d'inconvénients ultérieurs, tels que: irritations, abcès et autres malaises dérivés d'une implantation de ce genre.

Dans la deuxième exécution, il s'agit de deux pièces, l'une d'elles formée par une plaque adhésive (applicable à la paroi abdominale) et l'autre par un obturateur jetable fixé sous pression à ladite plaque, un obturateur qui est en matière plastique molle et souple, contient un filtre de charbon, et est emballé dans un film hydrosoluble qui se désintègre et permet que ledit obturateur s'élargisse pour éviter le passage des fèces. Il s'agit d'une réalisation compliquée, également ennuyeuse et susceptible de provoquer des difficultés chez certains patients.

Par ailleurs, il est connu par THE LANCET , Vol.II, No 8515, du 8 nov. 1986, pages 1062-1063 par Flemming Burchart et al., Londres, GB, un dispositif obturateur pour le contrôle d'iléocolostomies, dont l'office d'obturation est obtenu par la pression que son corps exerce sur les parois du colon lorsqu'il augmente de volume, une fois introduit dans le stomate, ce qui détermine un risque grave de produire une nécrose. Il s'agit d'un dispositif d'un seul usage qui ne peut être ni démonté ni nettoyé ni, encore moins, réutilisé. On connait par ailleurs ceux qui sont composés par une plaque de base, un bouchon pénétrant et des moyens de filtration par les brevets No EP-A-0037785 (Laboratoires Biotrol, S.A.) et DE-A-2634642 (McDonnell et al.). Ces dispositifs ont l'inconvénient qu'il est impossible que les usagers les réutilisent et ils ne peuvent être utilisés alternativement avec la poche courante car ils ne possèdent pas une base de support ambivalente.

Quant à l'adoption d'un filtre absorbant pour les gaz, on peut également signaler le brevet No EP-A-0 191646 (Craig Medical), qui permet de le brancher à toute poche d'ostomie par un boudin articulé et le fixer au moyen d'une feuille adhésive.Cette particularité est différente de l'invention car le filtre du dispositif est logé dans un couvercle articulé qui est fermé sous pression.

Pour éviter tous ces inconvénients, on a conçu le dispositif objet de la présente demande, qui comporte un couvercle extérieur, un anneau le retenant, un feuillet d'adhésif poreux, une rondelle de Karaya qui détermine la plaque de base de l'ensemble et un bouchon obturateur d'un profil déterminé et fixé audit couvercle. Comme on peut comprendre, il s'agit de la combinaison d'éléments partiellement nouveaux, à facile adaptation et libres des défauts propres aux modèles suscités.

Le présent brevet concerne également des améliorations introduites dans l'exécution citée au paragraphe précédent, qui consistent en doter ledit dispositif d'un moyen d'échappement des gaz des vents composé par une perforation passante pratiquée axialement dans la tige centrale qui émerge de la face intérieure du couvercle principal et qui est destiné à fixer le bouchon creux à forme d'une poire qui doit pénétrer dans le colon, en communiquant ladite perforation aussi bien avec l'intérieur dudit bouchon qu' avec une enceinte qui se ferme avec un couvercle auxiliaire et qui est destinée à contenir la pastille de carbone activé correspondante contre la mauvaise odeur, en pratiquant dans ledit couvercle auxiliaire et ladite pastille, ainsi qu'à la paroi du propre bouchon creux, plusieurs orifices aptes pour la circulation et l'expulsion desdits gaz de l'intérieur dudit intestin vers l'extérieur.

Pour une meilleure compréhension de la présente description, des feuilles de dessins sont jointes dans lesquelles , seulement à titre d'exemple et non limitatif, sont représentés deux cas pratiques d'exécution d'un dispositif aux caractéristiques générales exposées.

Dans lesdits dessins:
La Fig. 1 est une vue en coupe et éclatée des parties qui forment ledit dispositif obturateur.
La Fig. 2 correspond à la précédente, mais à présent avec lesdits éléments couplés et en position d'usage; et
La Fig. 3 montre, également en coupe, ce même dispostif appliqué à un patient.
Les fig. 4, 6 et 9 correspondent aux 1, 2 et 3, respectivement, mais à présent elles sont propres à l'exécution améliorée suscitée;
La fig. 5 est une vue en plan inférieure de la fig. 4;
La fig. 7 est un plan de la figure 6, et
La fig. 8 montre, en une plus grande échelle, un détail de la partie occupée par l'absorbeur d'odeurs de la réalisation des figures 4, 6 et 9.

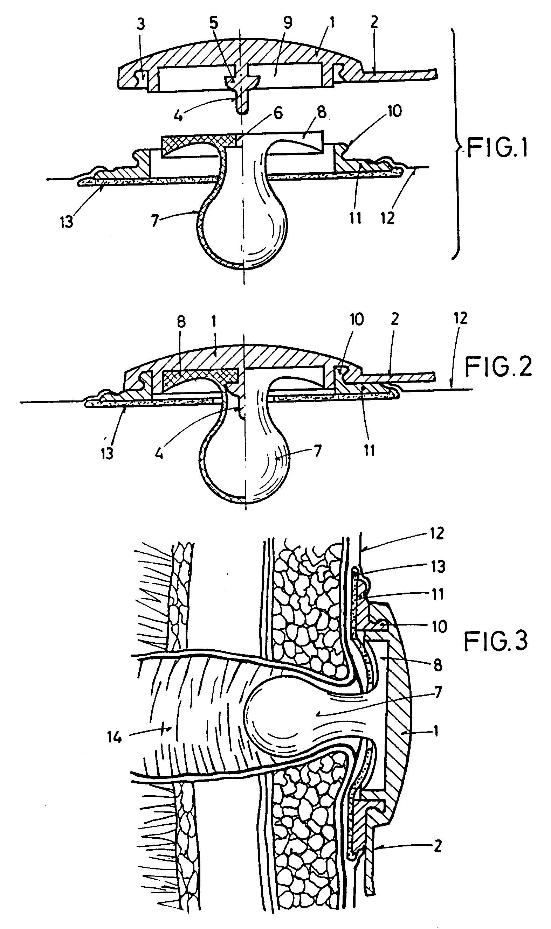

L'objet de cette demande est déterminée (Fig. 1 à 3) par un couvercle circulaire et légèrement bombé (1), en matière plastique, dotée d'un rebord latéral (2), d'une cavité annulaire d'accouplement (3) et une tige ou tenon central intérieur (4), muni d'un rebord d'accrochage (5), apte à y être introduite et y fixé sous pression dans le col étranglé (6) pratiqué dans un bouchon obturateur creux (7), à forme sensiblement d'une poire et portant un collier (8), qui est adossé au plafond (9) ou enfoncement qui entoure la tige (4). Cet obturateur (7-8) est en caoutchouc ou analogue et, une fois couplé au couvercle (1), il reste tel qu'il est montré dans la fig. 2.

L'ensemble de ces deux pièces est fixé, également sous pression et par l'introduction forcée dans la cavité annulaire (3), qui agit de femelle de fermeture, du bord (10) façonné en une pièce également annulaire (11), à laquelle sont collés un feuillet d'adhésif poreux (12) et la rondelle en caoutchouc Karaya® (13), qui compose la plaque de base de l'ensemble.

Dans le détail de la fig. 3 on voit comment est appliqué le dispositif et comment le bouchon obturateur (7) pénètre dans l'orifice du colon (14) en passant par l'ouverture de communication (anus contre nature) qui y est pratiquée.

Les deux composants (1) et (7-8) (couvercle et bouchon obturateur) se solidarisent par le biais du système d'accrochage central déterminé par la tige (4) et l'orifice (6). L'ensemble ainsi constitué (Fig. 2) peut être appliqué sous pression à l'anneau (11) uni à la paroi abdominale grâce à la pénétration du bord (10) (mâle) dudit anneau dans la cavité annulaire (3) (femelle) du couvercle (1). Dans la phase de fermeture, l'obturation obtenue ist totale (fig. 3) pour les fèces et les gaz et lorsque l'ouverture pour l'évacuation intéressera, il suffit de tirer ledit bouchon à travers le rebord (2), avec quoi l'orifice de l'anus restera libre pour le réglage, également sous pression, de la poche de colostomie appropriée.

La réalisation décrite jusqu'à ce moment (fig. 1 à 3) présente les particularités suivantes:
Comme on peut voir, les avantages que la réalisation exposée présente sont les suivants:
a) Il n'est pas nécessaire d'intervenir chirurgiquement dans la paroi abdominale pour y occlure un corps étranger (par exemple, l'aimant d'un des modèles sur le marché).
b) Toutes les pièces employées sont résistantes aux liquides naturels et l'obturateur, comme il est élastique, est adapté à tout diamètre de l'orifice de l'anus. De plus, la forme de poire de ce bouchon a l'avantage de contribuer à la retenue, à part la fixation au moyen du couplement extérieur (3-10) suscité.
c) L'ensemble peut être facilement nettoyé à fond, en décrochant simplement (1) et (7) pour la séparation.
d) Une fois ledit obturateur est séparé, on peut fixer à l'anneau (11) celui de la poche de colostomie usuelle pour recevoir les fèces lorsque cela intéressera.
e) Grande facilité de placement, longue durée et coût réduit.

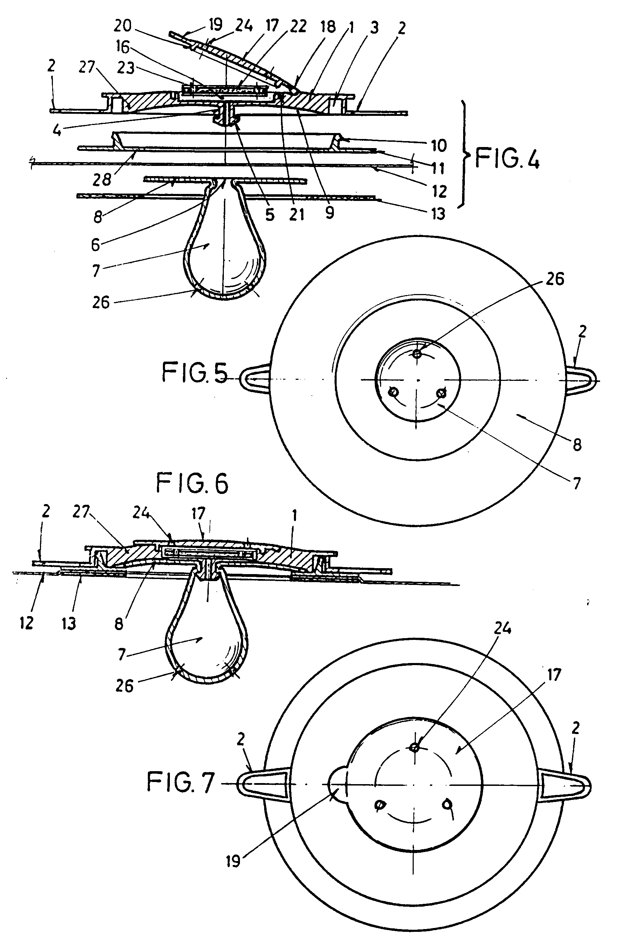

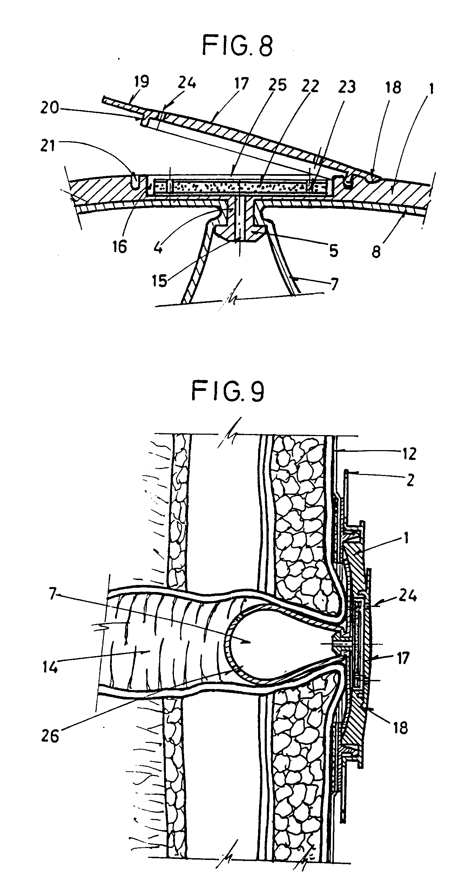

La réalisation perfectionnée qui va être décrite (fig. 4 à 9) comporte également le couvercle principal circulaire (1), en matière plastique, muni ue deux rebords latéraux (2) pour la manipulation, ainsi que le canal annulaire (3). Ce couvercle (1) présente la cavité voûtée (9) , d'où émerge la tige centrale (4) portant le rebord maintenant extrême (5) et un orifice passant (15), qui communique avec une enceinte circulaire (16), qui peut être fermée au moyen d'un couvercle auxiliaire (17), articulé à charnière par le point (18) et munie d'un rebord de manipulation (19) et d'une nervure mâle (20) qui, avec la gorge femelle (21), détermine une fermeture sous pression pour cette enceinte (16) dans laquelle est logée une pastille discoïdale de carbone activé (22), possédant des orifices (23). Le couvercle (17) se trouve également perforée par plusieurs orifices (24) (fig. 8) qui intercommunique l'enceinte (16) avec l'extérieur. Le gros de cette pastille (22) a été calculé de sorte que entre elle et le toit du couvercle (17) il reste une petite chambre de freinage (25) (fig. 8).

Au fond de la cavité voûtée (9) est appliquée et adapté un collier élastique (8) du bouchon à forme de poire creuse (7) qui, en plus d'être doté de la bouche étranglée (6) pour son accrochage sous pression avec la tige (4) (fig. 8), dispose de plusieurs orifices (26). Une fois l'accouplement réalisé, les deux composants (1) et (7) constituent une unité, de sorte que le bouchon (7) suit le couvercle (1) aussi bien lorsque l'anus contre nature pratiqué dans le colon (14) s'ouvre ou se ferme.

Le dispositif est complété par l'anneau (11) portant la nervure annulaire profilée (10) qui agit comme moyen d'accrochage mâle lorsqu'il pénètre forcé dans le canal (3). Dans cette position de réglage, le bord intérieur (27) du couvercle (1) vient incider sur le rebord intérieur (28) de cet anneau (11) en formant là une ligne étanche de fermeture.

La face extérieure de ce même anneau (11) reçoit la feuille adhésive (12), à laquelle est également fixée la rondelle plate (13), à extérieur également adhésif. Les éléments (12) et (13) sont ceux qui assurent l'adhérence de l'ensemble sur l'épiderme de la zone qui entoure l'orifice pratiqué dans le colon (14) (Fig. 9). Les points les plus importants à remarquer dans cette exécution largement améliorée sont les suivants:
a) Le couvercle (1) dispose d'une tige d'accrochage (4), perforée axialement pour permettre le passage des gaz (vents) vers l'enceinte (16) où ils perdent leur odeur lorsqu'ils traversent la pastille de carbone activé (22). La sortie vers l'extérieur est garantie par les perforations (23) de ladite pastille et celles (24) du couvercle (17).
b) Le couvercle articulé (17) permet de rechanger facilement la pastille de carbone activé (22), qui laisse passer les gaz qui, avant de sortir à l'extérieur, s'arrêtent un peu dans la chambre (25) pour un bon contact avec le carbone et assurer de cette façon la perte de la mauvaise odeur.
c) L'intérieur du bouchon (7) communique avec l'enceinte (16) à travers le passage (15) donc lesdits gaz provenant de l'intestin (14) doivent forcément sortir à l'extérieur en passant par la pastille (22) et en traversant le couvercle (17).
d) Le bouchon obturateur ou de fermeture proprement dit (7) dispose de plusieurs orifices (26) pour la circulation desdits gaz. Ces orifices (26), en nombre variable, sont pratiqués dans une ligne ou niveau qui ne peut jamais rester bouché par la paroi de l'intestin (14).
e) Le nombre d'orifices du couvercle (17), de la pastille (22) et du bouchon (7) est variable, mais ne sera jamais inférieur à deux, aux fins d'assurer que toujours, au minimum un d'eux, sera découvert pour la libre expulsion desdits gaz.
f) L'anus peut se fermer aussi bien avec le dispositif décrit qu'avec la poche de colostomie usuelle, selon les besoins de l'utilisateur. Durant le jour on emploie normalment ledit dispositif et pendant la nuit, comme l'excrétion ne peut pas être contrôlée, ladite poche est appliquée en permanence pour recueillir les fèces et les gaz.
g) Les composants (1) et (11) sont à nature semi dure (par exemple en matière plastique appropriée), tandis que les pièces (7), (12) et (13) sont molles (souples ou élastiques), dont la première est fabriquée avec un élastomère approprié (caoutchouc naturel ou synthétique). Le feuillet (12) est en papier adhésif poreux et la rondelle (13) est constituée par le caoutchouc de Karaya® habituel.

Il faut signaler que le diamètre intérieur de ladite rondelle (13) , qui, à ce jour, était découpée à la main selon l'étendue de la zone occupée par l'orifice de l'anus, est définie déjà à l'origine, c'est-à-dire qu'il ne faut faire aucune manipulation car tout le dispositif est livré dans diverses dimensions. Cela représente plusieurs avantages aussi bien pour le patient que pour le personnel médical.

La façon d'agir du dispositif décrit est facilment déduite de ce qui a été exposé, il suffit de signaler les avantages suivants: La fermeture est parfaite; la manipulation n'offre aucune difficulté; le patient peut utiliser l'obturateur pendant le jour avec l'assurance que les gaz (vents) qui pourraient se produire sortiront à l'extérieur sans aucune odeur désagréable, tandis que pendant la nuit (ou aussi pendant le jour si c'était nécessaire) il peut être remplacé par la poche normale collectrice de fèces; cette simplicité de fermeture et d'ouverture garantissent une hygiène totale de la zone intervenue et les matériaux employés dans le bouchon (18) ne produisent aucun malaise grâce à leur douceur.

Seront indépendants de l'objet de l'invention les matériaux, formes et dimensions des divers composants du dispositif obturateur dans ses deux versions décrites à condition que les variations qui seront introduites n'en affectent pas l'essentialité.

## Revendications

1. Dispositif obturateur pour le contrôle d'iléocolostomies, du type constitué par trois éléments principaux, le premier élément étant défini par un couvercle extérieur (1), le deuxième par un bouchon élastique creux (7), et le dernier par la base ou plaque usuelle (11) d'application à la face extérieure de la région abdominale et en entourant l'orifice ou anus, qui est caractérisé en ce que le couvercle (1) (qui est en matériau dur, tel qu'un plastique approprié) présente, sur la face qui doit se coupler au bouchon (7), une cavité annulaire (3) qui entoure un fond (9) au centre duquel apparait un tenon (4) muni d'un bord d'accrochage (5) destiné à collaborer avec un orifice (6) situé à une extrémité dudit bouchon, qui est en matière élastique (par exemple en caoutchouc), creux et à forme de poire et dispose d'un collier (8) qui s'adapte exactement audit fond (9) du couvercle (1), la base d'application étant déterminée par l'adhésif poreux conventionnel (12) et la rondelle usuelle de Karaya (R) (13).

2. Dispositif obturateur pour le contrôle d'iléocolostomies, selon la revendication 1, qui est caractérisé en ce que l'accouplement entre le couvercle et la pièce annulaire (11) qui compose une partie de la base du dispositif se fait par l'adaptation d'un bord mâle (10) de ladite pièce à l'intérieur de la cavité annulaire femelle dudit couvercle, le rebord (2) de ce dernier permettant de pouvoir retirer l'ensemble pour extraire le bouchon obturateur au moment où il faut appliquer la poche de colostomie.

3. Dispositif obturateur pour le contrôle d'iléocolostomies, selon les revindications précédentes, qui est caractérisé en ce que dans une exécution améliorée, il possède un moyen d'échappement des gaz des vents composé par une perforation passante (15) pratiquée axialement dans le tenon central (4) qui émerge de la face intérieure du couvercle principal (1) et qui est destiné à fixer le bouchon qui doit pénétrer dans le colon, cette perforation communiquant aussi bien avec l'intérieur dudit bouchon qu'avec une chambre (16) qui se ferme avec un couvercle auxiliaire (17) et qui est destinée à contenir une pastille de charbon actif (22), en pratiquant dans ledit couvercle auxiliaire et ladite pastille, ainsi qu'à la paroi du propre bouchon creux, plusieurs orifices (24-26) aptes pour la circulation et expulsion desdits gaz de l'intérieur de l'intestin vers l'extérieur.

4. Dispositif obturateur pour le contrôle d'iléocolostomies selon la revendication 3, qui est caractérisé en ce que le fond (9), normalement voûté, du couvercle principal (1), auquel est appliqué le collier (6) qui possède le bouchon creux cité, finit en un bord annulaire (27) qui incide, avec réglage étanche, sur un rebord intérieur (28) de l'anneau qui est fixé au corps du patient et qui, en plus de posséder le couplement langueté courant pour ce couvercle, définit maintenant un orifice à diamètre standard dans la zone de pose en entourant l'ouverture de l'anus.

5. Dispositif obturateur pour le contrôle d'iléocolostomies selon la revendication 3, qui est caractérisé en ce que le couvercle de la chambre qui loge la pastille de charbon actif (22) se trouve articulé à charnière et présente sur sa face intérieure des moyens de fermeture sous pression, composés, de préférence, par un jeu de nervure et rainure (20-21) pour un couplement langueté.

6. Dispositif obturateur pour le contrôle d'iléocolostomies selon les revendications 3 et 5 qui se caractérise en ce que la profondeur de la chambre (16) qui contient la pastille de charbon actif est telle par rapport à la grosseur de cette dernière que entre elle et le couvercle de fermeture (17) il se forme une petite chambre (25) de freinage prévue pour retenir un peu les gaz qui doivent sortir inodores à l'extérieur.

7. Dispositif obturateur pour le contrôle d'iléocolostomies selon les revendications 3, 5 et 6 caractérisé en ce que les orifices (23) qui traversent la pastille de charbon, le couvercle articulé (17) et la paroi du bouchon creux (7) sont en nombre non inférieur à deux, ceux dudit bouchon étant disposés dans un niveau qui ne peut pas être bouché par la paroi du colon une fois le dispositif obturateur y est introduit.

## Claims

1. Closure device for controlling ileo colostomies, of the type comprising three main components, the first component being defined by an outer cover (1), the second by a hollow elastic stopper (7), and the third by the normal base or plate (11) to be applied to the outer surface of the abdominal region surrounding the opening or anus, which is characterised in that the cover (1) (which is in a hard material, such as a suitable plastic) is provided on the surface to be attached to the stopper (7), with a ring-shaped cavity (3) surrounding a base (9) in the centre of which there appears a lug (4) having a hooking edge (5) intended to join with an opening (6) at one end of the said pear-shaped hollow stopper, which is in an elastic material (for example in rubber) and is provided with a collar (8) which accurately fits the said base (9) of the cover (1), the plate to be applied being fixed by the conventionally used porous adhesive (12) and the normal Karaya (R) washer (13).

2. Closure device for controlling ileo colostomies, according to claim 1, characterised in that the connection between the cover and the ring-shaped member (11) comprising part of the base of the device is formed by fitting a male rim (10) of the said member inside the female ring-shaped cavity of the said cover, the rim (2) of the latter permitting the whole unit to be withdrawn in order to withdraw the closure stopper when the colostomy pocket has to be attached.

3. Closure device for controlling ileo colostomies, according to the above claims, characterised in that in an improved embodiment, it is provided with a means of escape for wind gases consisting of a sliding perforation (15) contrived in an axial manner in the central lug (4) emerging from the inner surface of the main cover (1) which is intended to attach the stopper entering the colon, this perforation connecting both with the inside of the said stopper and with a chamber (16) which is closed with an auxiliary cover (17) and which is intended to contain an active charcoal tablet (22), by contriving in the said auxiliary lid and the said tablet, and also on the wall of the hollow stopper itself, several openings (24-26) for the purpose of circulating and ejecting the said gases from the inside of the intestine outwards.

4. Closure device for controlling ileo colostomies according to claim 3, characterised in that the base (9) which is normally arched, of the main cover (1), to which there is applied the collar (6) provided with the above mentioned hollow stopper, terminates in a circular rim (27) which has an angle of incidence with a watertight adjustment, onto an inner rim (28) of the ring which is attached to the patient's body and which, besides being provided with the tongued connection for this cover, now defines an opening of a standard diameter within the placement area by surrounding the opening of the anus.

5. Closure device for controlling ileo colostomies according to claim 3, characterised in that the cover of the chamber housing the active charcoal tablet (22) is articulated to the hinge and on its inner surface is provided with means of closure under pressure which preferably consist of a cooperating rib and groove (20-21) for a tongued connection.

6. Closure device for controlling ileo colostomies according to claims 3 and 5, characterised in that the depth of the chamber (16) containing the active charcoal tablet is such in relation to the thickness of the latter that between itself and the closure cover (17) a small retarding chamber (25) is provided in order to retain slightly the gases which ought to be odourless when they emerge.

7. Closure device for controlling ileo colostomies according to claims 3, 5 and 6 characterised in that the openings (23) crossing the charcoal tablet, the hinged cover (17) and the wall of the hollow stopper (7) are not less than two in number, those of the said stopper being arranged at a level which cannot be blocked by the wall of the colon once the closure device is inserted.

## Patentansprüche

1. Verschlußvorrichtung zur Kontrolle von Ileokolostomien, von der drei Hauptelemente aufweisenden Art, wobei das erste Element von einem Außenverschluß (1) gebildet wird, das zweite von einem hohlen elastischen Stopfen (7) und das letzte von dem üblichen Basisteil oder der Platte (11), die auf der Außenseite des Bauchbereiches anliegt und die Afteröffnung umgibt, **dadurch gekennzeichnet**, daß der Verschluß (1) (der aus einem harten Material wie einem geeigneten Kunststoff besteht) auf der mit den Stopfen (7) zu verbindenden Seite eine ringförmige Vertiefung (3) aufweist, die einen Boden (9) umgibt, in dessen Mitte sich ein Zapfen (4) mit einem Befestigungsrand (5) befindet, der mit einer an einem Ende des Stopfens befindlichen Öffnung (6) des Stopfens zusammenwirkt, der aus elastischem Material (z.B. Gummi) besteht, hohl ist, die Form einer Birne hat und einen Rand (8) aufweist, der genau dem Boden (9) des Verschlusses (1) angepaßt ist, und daß die Platte durch den herkömmlichen porösen Klebestreifen (12) und den üblichen Karayaring (R) (13) gebildet wird.

2. Verschlußvorrichtung zur Kontrolle von Ileokolostomien nach Anspruch 1, **dadurch gekennzeichnet**, daß die Verbindung zwischen Verschuß und ringförmigem Basisteil (11), das einen Teil des Bodens der Vorrichtung bildet, durch die Einpassung eines Außenrandes (10) des Basisteils in die ringförmige Innenvertiefung des Verschlusses hergestellt wird, an dessen Randleiste (2) man die zusammengefügten Teile zurückziehen kann, wenn der Verschlußstopfen beim Anbringen des Auffangbeutels herausgezogen wird.

3. Verschlußvorrichtung zur Kontrolle von Ileokolostomien nach den vorstehenden Ansprüchen, **dadurch gekennzeichnet**, daß bei einer verbesserten Ausführung eine Vorrichtung zum Abführen der Darmgase vorgesehen ist, die aus einer Durchführungsöffnung (15) besteht, welche axial in dem aus der Innenseite des Hauptverschlusses (1) herausragenden Mittelzapfen (4) ausgebildet ist, der den in das Kolon ragenden Stopfen festhält, wobei die Durchgangsöffnung sowohl mit dem Inneren des Stopfens als auch mit einer Kammer (16) in Verbindung steht, welche mit einem Hilfsverschluß (17) verschlossen wird und zur Aufnahme einer Aktivkohletablette (22) dient, wobei im Hilfsverschluß und der Tablette, ebenso wie in der Wand des eigentlichen hohlen Stopfens mehrere Löcher (24-26) vorgesehen sind, durch die die Gase hindurchtreten und aus dem Darminnern nach außen gelangen können.

4. Verschlußvorrichtung zur Kontrolle von Ileokolostomien nach Anspruch 3, **dadurch gekennzeichnet**, daß der normalerweise gewölbte Boden (9) des Hauptverschlusses (1), an dem der Rand (6) des hohlen Stopfens anliegt, in einem ringförmigen Rand ausläuft (27), der unter Abdichtung an einem Innenrand (28) des am Körper des Patienten befestigten Rings stößt und der nicht nur die übliche Eingriffsverbindung für diesen Verschluß besitzt, sondern nunmehr eine Öffnung mit Standarddurchmesser im Anlagebereich bildet, indem er die Afteröffnung umgibt.

5. Verschlußvorrichtung zur Kontrolle von Ileokolostomien nach Anspruch 3, **dadurch gekennzeichnet**, daß der Verschluß der Kammer, in der sich die Aktivkohletablette (22) befindet, mittels eines Gelenkes schwenkbar ist und an seiner Innenseite über Druckverschlußmittel verfügt, die vorzugsweise aus einer Rippe und der dazugehörigen Rille (20 und 21) für eine Eingriffsverbindung bestehen.

6. Verschlußvorrichtung zur Kontrolle von Ileokolostomien nach den Ansprüchen 3 und 5, **dadurch gekennzeichnet**, daß die Tiefe der Kammer (16), welche die Aktivkohletablette enthält, bezüglich deren Größe so bemessen ist, daß zwischen ihr und dem Verschluß (17) eine kleine Rückhaltekammer (25) verbleibt, welche die Gase, die geruchlos nach außen gelangen sollen, ein wenig zurückhalten soll.

7. Verschlußvorrichtung zur Kontrolle von Ileokolostomien nach den Ansprüchen 3, 5 und 6, **dadurch gekennzeichnet**, daß mindestens zwei durch die Kohletablette, den Gelenkverschluß (17) und die Wand des hohlen Stopfens (7) führende Löcher (23) vorgesehen sind, wobei die Löcher des Stopfens auf einer solchen Höhe angeordnet sind, daß sie nach dem Einführen der Verschlußvorrichtung nicht durch die Kolonwand verstopft werden können.
